# EUROPEAN PATENT APPLICATION

(11) **EP 4 285 813 A1**
(43) Date of publication of application: **06.12.2023**
(21) Application number: 21923354.1
(22) Date of filing: 29.04.2021
(51) Int. Cl.: A61B 5/00, A61B 8/00, A61B 8/08

(54) **PHOTOACOUSTIC DETECTION SYSTEM COMBINED WITH TRANSPARENT ULTRASONIC SENSOR**

(30) Priority: 01.02.2021 KR 20210014148
(71) Applicant: Postech Academy-Industry Foundation, Gyeongsangbuk-do, 37673 (KR)
(72) Inventor: KIM, Chul Hong, Pohang-si Gyeongsangbuk-do 37673 (KR); PARK, Jeong Woo, Daegu 42034 (KR); KIM, Hyung Ham, Pohang-si Gyeongsangbuk-do 37673 (KR); PARK, Byul Lee, Gwangju 61745 (KR); HAN, Moongyu, Pohang-si Gyeongsangbuk-do 37676 (KR)
(74) Representative: Nederlandsch Octrooibureau
(86) International application number: PCT/KR2021/005468
(87) International publication number: WO 2022/163944

(57) **Abstract**

Disclosed is a photoacoustic detector system combined with transparent ultrasonic sensors.

A photoacoustic detector system combined with transparent ultrasonic sensors according to an embodiment includes: a light source from which an optical oscillation device generates light appropriate for generating a photoacoustic phenomenon; a light transmission system which transmits the light generated from the light source to a probe side; a probe which accommodates an optical system and the transparent ultrasonic sensor therein; the optical system which adjusts the size, focus, path, etc., of the light generated from the light source; the transparent ultrasonic sensor which detects an ultrasonic wave generated in an observation area in response to light irradiated to the observation area according to a photoacoustic effect, and is made of a light-transmitting material to transmit the light generated from the light source; a data collection device which collects photoacoustic data including the ultrasonic wave detected by the transparent ultrasonic sensor; and a data detection device which analyzes the photoacoustic data and determines whether a specific material is detected in the observation area.

## Description

### [Technical Field]

The present invention relates to a photoacoustic detector system combined with transparent ultrasonic sensors, and more particularly, to a photoacoustic detector in which an axis linking an ultrasonic sensor and an ultrasonic generation point and an axis linking a light source and a light irradiation point coincide with each other to obtain a high SNR and acquire an ultrasonic image of a lesion such as a sentinel lymph node, etc.

### [Background Art]

The presence or absence of lymph node metastasis of a malignant tumor is a major factor in determining the patient's survival rate. In particular, since the malignant tumor first metastasizes to the 'sentinel lymph node (SLN)', accurate detection of SLN and histological examination are very important for the patient's prognosis. Representative cancers for which SLN biopsy is mandatory include breast cancer and cutaneous melanoma, and SLN biopsy is also increasing in oral cancer and gastric cancer.

Current SLN biopsy involves injecting radioactive material, detecting the position of SLN with a gamma ray detector (radioscope) during surgery, and undergoing excision. The gamma ray detector (radioscope) is held and controlled directly by an operator.

However, in the SLN biopsy using the radioscope, there is a risk of radiation exposure to both the subject and operator due to the nature of injecting radioactive material into the tissue of the subject during the lymph node biopsy process. In addition, since the price of the radioscope is high and the radioscope needs to be used in a separate special space, it is possible only in very limited hospitals. Moreover, considerable costs are required for disposal of the radiation-related material after use.

On the other hand, as an alternative to solving the problem of exposure risk due to radiation examination of SLN tissue examination using such a radioscope, a photoacoustic detector using a photoacoustic sensor has emerged instead of gamma rays.

A photoacoustic phenomenon is a phenomenon in which an object absorbs light and generates ultrasonic waves when light of a specific wavelength band is irradiated onto an object. The photoacoustic detector is a device using this photoacoustic phenomenon.

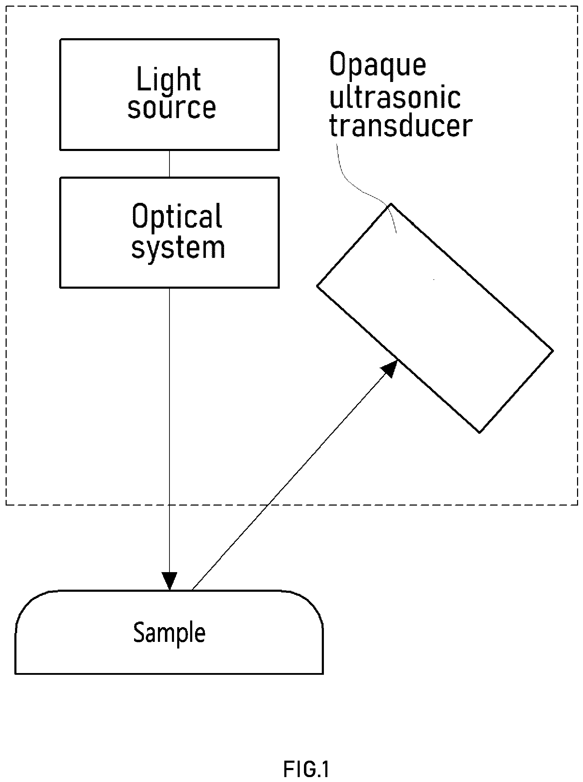

FIG. 1 is a diagram illustrating an example of a photoacoustic detector according to the prior art.

The photoacoustic detector is configured to largely include a light source for irradiating light of a specific wavelength band to an object, and an ultrasonic sensor (or having the same meaning as an ultrasonic transducer) which detects an ultrasonic wave generated from an object to generate an ultrasonic image using the ultrasonic wave generated from the light irradiated to the object.

This photoacoustic detector combines the advantages of a high-resolution optical system and an ultrasound system that can penetrate relatively deep tissues under the skin compared to other medical imaging equipment to detect various tissues or organs (cancer tissues, blood vessels, contrast agents, etc.) with high resolution even in a deep space in a biological tissue.

However, in the case of the photoacoustic detector according to the prior art, since the ultrasonic sensor is opaque, light emitted from the light source may not pass through the ultrasonic sensor. Accordingly, it was impossible to arrange an optical system of a light source requiring a transparent medium and an opaque ultrasonic sensor on the same axis.

As a result, as illustrated in FIG. 1, the photoacoustic detector in which the light source and the ultrasonic sensor are disposed at an angle inclined within the photoacoustic detector have been proposed.

This conventional photoacoustic detector has the following problems.
1. The large size of the photoacoustic detector prevents the photoacoustic detector from approaching the target and causes inconvenience in use.
2. Although it is known that a high signal-to-noise ratio (SNR) is obtained when the light source and the ultrasonic sensor point to the exact same plane, signal attenuation and artifacts occur due to the positional difference between the ultrasonic sensor and the light source, so detection sensitivity decreases.

Thus, in order to obtain a high SNR, a photoacoustic SLN detector in which the axis linking the ultrasonic sensor and the ultrasonic generation point coincides with the axis linking the light source and the light irradiation point is required.

### [Disclosure]

### [Technical Problem]

The present invention was conceived in response to the above request, and has been made in an effort to provide a photoacoustic detector combined with transparent ultrasonic sensors in which an axis linking an ultrasonic sensor and an ultrasonic generation point and an axis linking a light source and a light irradiation point coincide with each other to obtain a high SNR and acquire an ultrasonic image of a lesion such as a sentinel lymph node, etc.

### [Technical Solution]

In order to solve the problem, a photoacoustic detector system combined with transparent ultrasonic sensors according to an embodiment includes: a light source from which an optical oscillation device generates light appropriate for generating a photoacoustic phenomenon; a light transmission system which transmits the light generated from the light source to a probe side; a probe which accommodates an optical system and the transparent ultrasonic sensor therein; the optical system which adjusts the size, focus, path, etc., of the light generated from the light source; the transparent ultrasonic sensor which detects an ultrasonic wave generated in an observation area in response to light irradiated to the observation area according to a photoacoustic effect, and is made of a light-transmitting material to transmit the light generated from the light source; a data collection device which collects photoacoustic data including the ultrasonic wave detected by the transparent ultrasonic sensor; and a data detection device which analyzes the photoacoustic data and determines whether a specific material is detected in the observation area.

At this time, the light source may be at least one light oscillation device selected from the group at least including an optically mediated resonator laser (OPO laser), a liquid die laser, an LED, an LD, a solid die laser, and an alexandrite laser.

In addition, the light transmission system may include at least one of an optical fiber and an articulated arm.

In addition, the optical system may at least include a beam expander, a mirror, and a lens.

In addition, the transparent ultrasonic sensor may be any one of a focus type and a non-focus type.

In addition, the transparent ultrasonic sensor may be a single transparent ultrasonic sensor element.

In addition, the transparent ultrasonic sensor may be constituted by an array of a plurality of transparent ultrasonic sensor elements.

In addition, a path axis of the light and a path axis of the ultrasonic wave may be formed parallel to each other.

In addition, the photoacoustic detector system may further include a wavelength conversion system which changes the wavelength of the light.

In addition, the photoacoustic detector system may further include a medium capable of transmitting the ultrasonic wave between the transparent ultrasonic sensor and the observation area.

In addition, the data collection device may receive the photoacoustic data by being wired or wirelessly connected to the transparent ultrasonic sensor.

### [Advantageous Effects]

Using the present invention is used, there is an effect that a photoacoustic detector system combined with transparent ultrasonic sensors can be implemented in which an axis linking an ultrasonic sensor and an ultrasonic generation point and an axis linking a light source and a light irradiation point coincide with each other to obtain a high SNR and acquire an ultrasonic image of a lesion such as a sentinel lymph node, etc.

Further, using the present invention is used, there is an effect that since radioactive materials are not used, it is possible to reduce costs as well as to enhance the safety of the operator and the subject unlike the radioscope.

In addition, using the present invention, it is possible to reduce the size of the photoacoustic detection probe, which was a limitation of the conventional photoacoustic technology, by using a transparent ultrasonic sensor. This is suitable for the photoacoustic detector that the operator must hold and use by hand, and has an effect of increasing detection accuracy by facilitating access of the probe to a substance to be detected.

In addition, using the present invention, signal attenuation and artifacts due to the difference in the position of the ultrasonic sensor and the light source do not occur, so the detection sensitivity increases, and the process of matching the ultrasonic axis and the axis of the light source, which depended on the user's experience, is not required, and as a result, there is an effect of increasing detection reliability.

In addition, using the present invention, photoacoustic detectors can be manufactured in various sizes as needed, so that there is an effect of implementing photoacoustic detectors having usability suitable for various body parts.

### [Description of Drawings]

FIG. 1 is a diagram illustrating a photoacoustic detector according to the prior art;
FIG. 2 is a diagram simply illustrating a measurement principle of a photoacoustic detector combined with transparent ultrasonic sensors;
FIG. 3 is a block diagram illustrating an example of the photoacoustic detector combined with transparent ultrasonic sensors;
FIG. 4 is a diagram illustrating various modified embodiments of a probe end of the photoacoustic detector combined with transparent ultrasonic sensors illustrated in FIG. 3;
FIG. 5 is a diagram illustrating a photoacoustic detector system including the photoacoustic detector combined with transparent ultrasonic sensors illustrated in FIG. 3; and
Figure 6 is an exploded perspective view showing a transparent ultrasonic sensor according to an embodiment.

### [Best Mode]

Hereinafter, a photoacoustic detector system combined with transparent ultrasonic sensors according to the present invention will be described in detail with reference to the drawings.

In describing the present invention, if it is determined that adding a detailed description of a technology or configuration already known in the field may obscure the gist of the present invention, it will be partially omitted from the detailed description. In addition, the terms used in this specification are terms used to properly express the embodiments of the present invention, which may vary depending on people or customs related to the field. Accordingly, definitions of the terms need to be made based on contents throughout this specification.

The terms used herein is for the purpose of describing specific embodiments only and are not intended to be limiting of the present invention. The singular forms used herein include plural forms as well, if the phrases do not clearly have the opposite meaning. A meaning "including" used in the specification means that a specific feature, region, integer, step, operation, element and/or component is embodied and other specific features, regions, integers, steps, operations, elements, components, and/or groups are not excluded.

In addition, a transparent ultrasonic sensor, a transparent ultrasonic transducer, and a transparent ultrasonic transducer (TUT) all refer to the same subject in this specification unless otherwise specified.

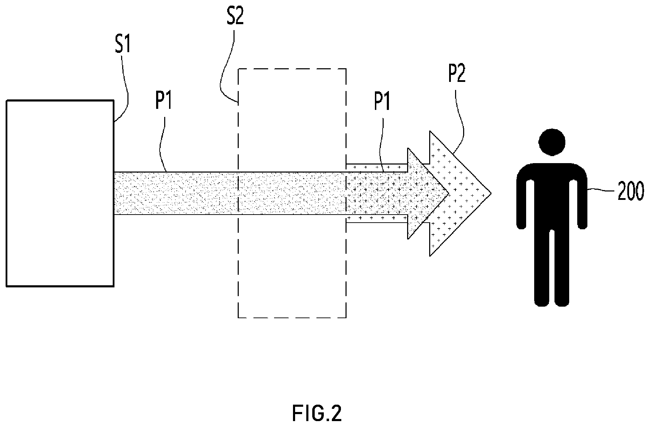

FIG. 2 is a diagram simply illustrating a measurement principle of a photoacoustic detector combined with transparent ultrasonic sensors.

In the photoacoustic detector combined with transparent ultrasonic sensors, a light source module S1 and a transparent ultrasonic sensor S2 are arranged in a row so that a path of light output from the light source module S1 and a path of an ultrasonic signal output from the transparent ultrasonic sensor S2 are parallel to each other.

That is, a light emission surface of the light source module S1 and an incident surface of the transparent ultrasonic sensor S2 are parallel to each other.

Thus, an optical path P1 finally incident on an object 200 and an ultrasonic path P2 of the transparent ultrasonic sensor S2 may be the same as or parallel to each other.

In this way, as the optical module S1 is located at the rear of the transparent ultrasonic sensor or on the same path as the path P2 of the transparent ultrasonic sensor, image acquisition of the object 200 located in front of the transparent ultrasonic sensor S2 becomes possible.

In this case, signals for the same position of the same object 200 are obtained without distortion of signals or light output from the optical ultrasonic sensor S2 and the optical module S1, so that an accurate image may be obtained.

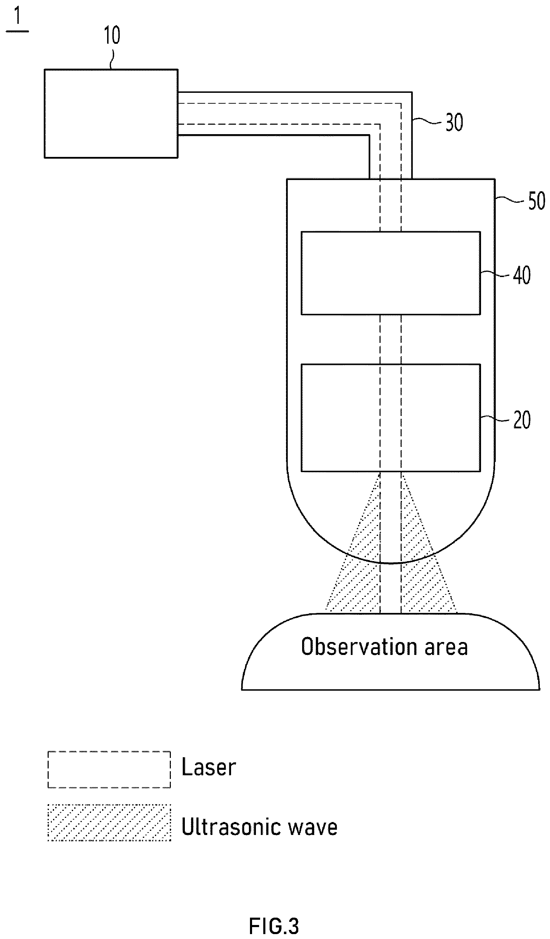

FIG. 3 is a block diagram illustrating an example of the photoacoustic detector combined with transparent ultrasonic sensors.

As illustrated in FIG. 3, the photoacoustic detector 1 combined with transparent ultrasonic sensors is configured to include a light source 10, a light transmission system 30, and a probe 50, and the probe 50 further includes an optical system 40 and a transparent ultrasonic sensor 20 again.

The light source 10 as a light oscillation device widely includes various light oscillation devices capable of generating light suitable for generating a photoacoustic phenomenon, such as an optically mediated resonator laser (OPO laser), a liquid die laser, an LED, an LD, a solid die laser, and an alexandrite laser.

The light transmission system 30 is a light transmission path system that transmits light generated by the light source 10 to the probe 50. In general, an optical fiber or an articulated arm is used, but the light transmission system is not limited only thereto.

The probe 50 is a part that packages and houses the optical system 40 and the transparent ultrasonic sensor 20 so that an operator may easily irradiate light to an observation area of a person to be operated.

The optical system 40 is used to adjust the size, focus, path, etc. of light generated by the light source 10, and various types of beam expanders, mirrors, and lenses may be used.

The transparent ultrasonic sensor 20 is an ultrasonic sensor made of light-transmitting elements so that the light generated by the light source 10 may be transmitted and irradiated to the observation area of the person to be operated. In addition, according to a photoacoustic effect, when ultrasonic waves are generated in the observation area in response to the light irradiated to the observation area, the transparent ultrasonic sensor 20 serves to sense the ultrasonic waves. As the transparent ultrasonic sensor 20, focused type and unfocused type transparent ultrasonic sensors of various frequency bands may be widely used. In addition, an ultrasonic sensor of a single element or an array element may be used depending on the purpose and necessity.

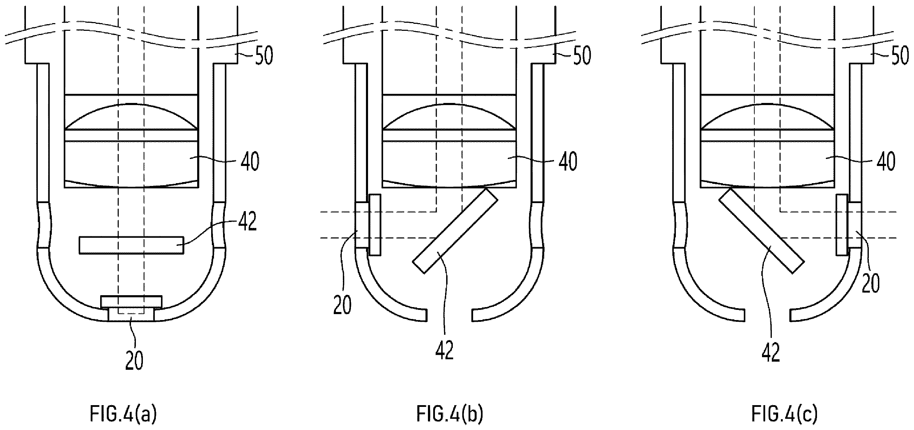

FIG. 4 is a diagram illustrating various modified embodiments of a probe end of the photoacoustic detector combined with transparent ultrasonic sensors illustrated in FIG. 3.

As illustrated in FIG. 4, the probe 50 allows the optical path and the ultrasonic path to be adjusted at the end of the probe 50 by placing a plano mirror 42 on the optical path and the ultrasonic path.

For example, in FIG. 4(a), an angle between the transparent ultrasonic sensor and an optical axis becomes 0°. As illustrated in FIG. 4(b), if the angle of the transparent ultrasonic sensor and the optical axis is adjusted so that the angle of the plano mirror 42 is -45°, the observation area of the person to be operated may be placed on the left side of the probe 50. Similarly, if the angle of the transparent ultrasonic sensor and the optical axis is adjusted so that the angle of the plano mirror 42 is +45 ° as illustrated in FIG. 4(c), the observation area of the person to be operated may be placed on the right side of the probe 50.

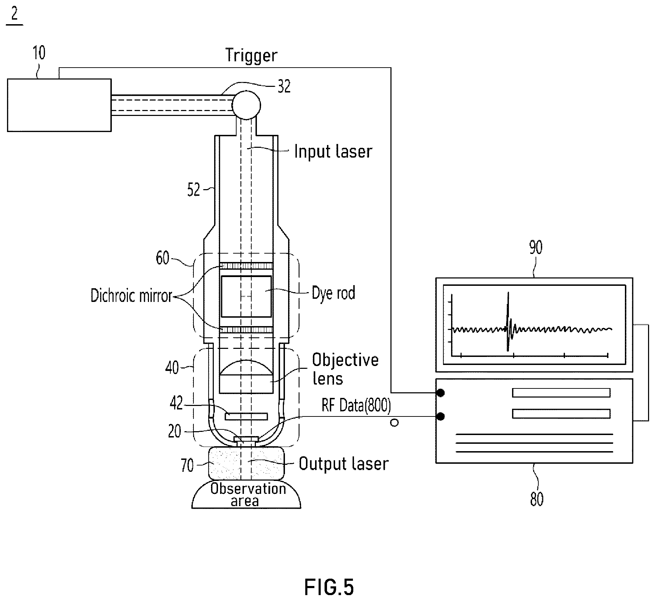

FIG. 5 is a diagram illustrating a photoacoustic detector system including the photoacoustic detector combined with transparent ultrasonic sensors illustrated in FIG. 3.

As illustrated in FIG. 5, the photoacoustic detector system 2 is configured to include a light source 10, an articulated arm 32, a photoacoustic detector case 42, a wavelength converter 60, an optical system 40, a transparent ultrasonic sensor 20, a medium 70, a data collection device 80, and a detection device 90.

The light source 10 as the light oscillation device widely includes various light oscillation devices capable of generating light suitable for generating a photoacoustic phenomenon, such as the optically mediated resonator laser (OPO laser), the liquid die laser, the LED, the LD, the solid die laser, and the alexandrite laser as mentioned above. At this time, a 532 nm wavelength laser mainly using Nd:YAG is used as a pump laser.

The articulated arm 32 as a passage for transmitting light may be used instead of an optical fiber or other light transmission system.

The photoacoustic detector case 52 corresponds to the probe 50 mentioned above. The laser oscillated by the light source 10 is transmitted to the inside of the photoacoustic detector case 52 through the articulated arm 32. The photoacoustic detector case 52 includes a wavelength changing system 60 and an optical system 40. In the case of the photoacoustic detector system 2 according to the present invention, the photoacoustic detector case 52 may be miniaturized to a level suitable for use by an operator holding the photoacoustic detector case 52 with one hand by making the optical path coincide with the ultrasonic path.

The wavelength conversion system 60 is a component for changing the wavelength of the pump laser. There are several methods for changing the wavelength of light, and in this embodiment, the wavelength of the laser light oscillated at 532 nm from the light source 10 is changed to 650 nm using a dye rod as the wavelength converter 60.

The optical system 40 is a component that changes a traveling path of the optical axis. As mentioned above, the optical system 40 is used to adjust the size, focus, path, and the like of the light generated by the light source 10, and a beam expander, a mirror, a lens, and the like may be variously used. By using the optical system 40, it is possible to position the transparent ultrasonic sensor 20 not only in front but also at various angles, enabling more efficient detection. Since the optical system 40 is not an essential element, the optical system 40 may be omitted in some cases.

The transparent ultrasonic sensor 20 is the ultrasonic sensor made of light-transmitting elements so that the light generated by the light source 10 may be transmitted and irradiated to the observation area of the person to be operated as described above. In addition, according to a photoacoustic effect, when ultrasonic waves are generated in the observation area in response to the light irradiated to the observation area, the transparent ultrasonic sensor 20 serves to sense the ultrasonic waves. As the transparent ultrasonic sensor 20, focused type and unfocused type transparent ultrasonic sensors of various frequency bands may be widely used. In addition, an ultrasonic sensor of a single element or an array element may be used depending on the purpose and necessity.

The medium 70 serves to transmit ultrasonic waves from the observation area to the transparent ultrasonic sensor 20 with high efficiency. The medium 70 may include various materials known to have properties capable of transmitting ultrasonic waves, such as water, ultrasonic gel, or ultrasonic pad.

The data collection device 80 is a device that collects photoacoustic data from the observation area input to the transparent ultrasonic sensor 20.

The data collection device 80 receives data from the transparent ultrasonic sensor 20 through wire or wireless.

The detection device 90 determines whether a specific substance is detected in the observation area by analyzing data from the observation area collected by the data collection device 80.

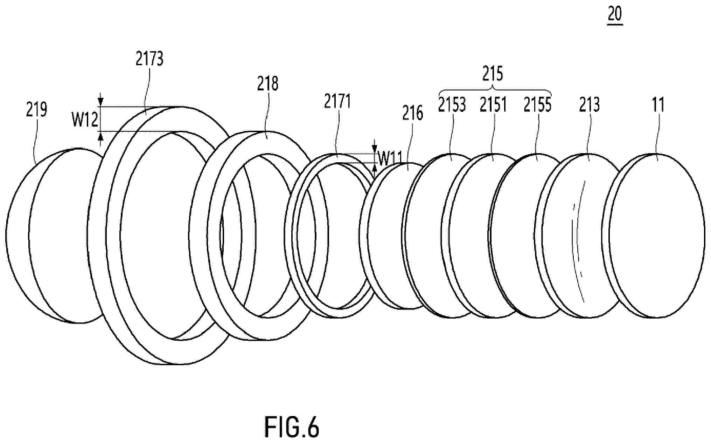

FIG. 6 is an exploded perspective view showing a transparent ultrasonic sensor according to an embodiment.

The transparent ultrasonic sensor 20 illustrated in FIG. 6 has a circular shape having a circular planar shape, but is not limited thereto.

As shown in FIGS. 2 to 4, the transparent ultrasonic sensor 20 according to an embodiment of the present disclosure may include, from the right side, a protective layer 211, an acoustic lens unit 213 located behind the protective layer 211, a piezoelectric unit 215 located behind the matching unit 213, first and second housings 2171 and 2173 connected to the piezoelectric unit 215, a rear layer 216 located behind the piezoelectric unit 215, an insulating unit 218 located between the first and second housings 2171 and 2173, and a correction lens unit 219 located behind the second housing 2173.

The protective layer 211 is to physically and electrically protect the transparent ultrasonic sensor 20 and to reduce a difference in acoustic impedance with a medium to which an ultrasonic signal is to be irradiated, that is, the sample A. Accordingly, the protective layer 211 has a protective function and may operate as a matching layer performing acoustic impedance matching between a liquid (e.g., water) and a living body.

The protective layer 211 may be formed of a transparent material. For example, the protective layer 211 may contain a transparent polymer, parylene.

In this example, the acoustic impedance of the protective layer 211 may be about 2.84 Maryl.

The protective layer 211 may be located on front and side surfaces of the piezoelectric unit 21 and on a side surface of the second housing 2173 located at the edge of the transparent ultrasonic sensor 20.

Accordingly, the protective layer 211 may eventually constitute the front and side surfaces of the transparent ultrasonic sensor 20.

The matching unit 213 located behind the protective layer 211 is to reduce a difference in acoustic impedance with a medium, i.e., the sample A, to which an ultrasonic signal generated by the piezoelectric unit 215 is to be irradiated.

That is, when an ultrasonic signal is generated by the operation of the piezoelectric unit 215, in order to efficiently transmit the ultrasonic signal in water, biological tissue, or a medium, other than air, the acoustic impedance of the corresponding medium should be adjusted as much as possible to minimize loss.

Each acoustic lens of the matching unit 213 of the present example may be a focused type using an acoustic lens capable of focusing light and ultrasonic signals.

As described above, since the matching unit 213 has a focus adjustment function, the ultrasonic signal reflected by the sample A and incident on the transparent ultrasonic sensor 20 is accurately focused on a desired position of the piezoelectric unit 215.

Accordingly, a focus of an ultrasound image obtained by the ultrasonic signal output from the piezoelectric unit 215 is adjusted by the focus adjustment function of the matching unit 213, so that a clear ultrasound image may be obtained.

Accordingly, the clarity of the image obtained by the operation of the transparent ultrasonic sensor 20 may be improved, so that a clear image may be obtained for a desired part of the sample A irradiated with the ultrasonic signal.

In addition, since the matching unit 213 uses an acoustic lens, a curve of the surface may be constant and the transparency of the surface may be improved, thereby reducing the loss of the ultrasonic signal when the ultrasonic signal irradiated to the sample A or reflected from the sample A is transmitted and received.

In addition, if necessary, an additional transmission or blocking layer may be formed on the matching unit 213 to transmit or block only a signal of a desired wavelength band.

The acoustic lens provided in the matching unit 213 may be formed of at least one of transparent glass, transparent epoxy, and transparent silicone.

Such an acoustic lens may be selected according to the function of the acoustic lens.

For example, when the acoustic lens functions as a matching layer performing an acoustic impedance matching function, if a piezoelectric material provided in the piezoelectric unit 215 is not in the form of a polymer, such as PVDF or PVDF-TrFE, It may be more preferable that the acoustic lens is formed of glass.

That is, when the piezoelectric material is formed of lithium niobite (LNO) or PMN-PT, acoustic impedance is as high as 30 to 40 Mrayl, but in the case of glass, acoustic impedance is as low as 210 to15 Mrayl, which is a numerical value facilitating matching of acoustic impedance and the transparency is very good, and thus, when the piezoelectric material is not in the form of a polymer, the acoustic lens may be formed of glass.

However, in a case in which the matching layer performing the acoustic impedance matching function has already been manufactured, the acoustic lens may be formed of transparent epoxy or transparent silicone.

That is, if a matching layer (about 7 to 20 Mrayl) performing a matching function already exist between a piezoelectric material having acoustic impedance of about 30 to 40 Mrayl and a biological tissue or water having acoustic impedance of about 21 to 2 Mrayl, a separate acoustic impedance matching operation is unnecessary, so epoxies or silicones (about 21 to 3 Mrayl) having acoustic impedance similar to that of biological tissue or water are appropriate. That is, since the acoustic impedance of epoxies and silicones have acoustic impedance almost similar to those of biological tissue or water, separate acoustic impedance matching is unnecessary.

In addition, considering the speed of sound and the speed of sound with respect to a material of the acoustic lens, a curvature of a curved surface of the acoustic lens and whether the acoustic lens is concave or convex may be determined.

For example, when the acoustic lens is formed of glass, an optical lens may be used. In this case, since the speed of light of glass is higher than that of water, the acoustic lens may be designed in a concave shape, such as a plano-concave.

When the acoustic lens is formed of transparent epoxy, a polishing process should be performed on a primarily manufactured acoustic lens to improve transparency as much as possible to finally complete the acoustic lens. As such, even when the acoustic lens is formed of epoxies, the epoxies have a faster speed of light than water, so the acoustic lens may also be manufactured in a plano-concave shape.

Even when the acoustic lens is formed of transparent silicone, like the case of epoxies, a separate polishing process should be performed to improve a finished acoustic lens as much as possible. In this case, since silicones have a speed of light lower than water, the acoustic lens may be manufactured in a convex shape, such as a plano-convex shape, unlike the cases of glass and epoxies. As such, when the acoustic lens is manufactured in a plano-convex shape, the acoustic lens may have a function of collecting light.

The piezoelectric unit 215 may include a piezoelectric layer 2151 and first and second electrode layers 2153 and 2155 located on the rear and front surfaces of the piezoelectric layer 2151, respectively.

The piezoelectric layer 2151 is a layer in which a piezoelectric effect and a converse piezoelectric effect takes place, and may contain a piezoelectric material that is at least one of lithium niobite (LNO), PMN-PT, PVDF, and PVDF-TrFE as described above.

A electromechanical coupling coefficient of LNO is very high as about 0.49, so the electromechanical energy conversion efficiency is very good.

In addition, since LNO has a low dielectric permittivity, when the piezoelectric layer 2151 is formed of LNO, the transparent ultrasonic sensor may be suitable for a large aperture single element transducer having a large opening.

In addition, since LNO has a high Curie temperature, LNO may withstand well even at a high temperature, so that the transparent ultrasonic sensor 20 having good heat resistance may be developed.

In addition, when the piezoelectric layer 2151 is formed of LNO, a single element ultrasonic sensor having a center frequency of 210 to 400 MHz may be easily developed.

When the piezoelectric layer 2151 contains PMN-PT, the piezoelectric performance (d33 ~ 21500-2800 pC/N) and electromechanical coupling coefficient (k>0.9) of PMN-PT are very high, so that the performance of the transparent ultrasonic sensor 20 may be improved.

Unlike LNO, PMN-PT has a high dielectric constant, so that the transparent ultrasonic sensor 20 suitable for a small aperture single or array ultrasonic sensor may be developed.

In addition, when the piezoelectric layer 2151 contains at least one of PVDF and PVDF-TrFE, the piezoelectric layer 2151 may have the following characteristics.

PVDF and PVDF-TrFE have the form of a polymer film, and it is possible to manufacture a piezoelectric layer 2151 that is flexible and stretchable, thereby reducing the thickness of the piezoelectric layer 2151 and it may be possible to manufacture the transparent ultrasonic sensor 20 for a signal of a high frequency band of about 2100 MHz by the reduced thickness.

In addition, PVDF and PVDF-TrFE have a relatively low electromechanical coupling coefficient and high receiving constant, have a wider bandwidth compared to other piezoelectric materials, and may be easy to manufacture in either a single device or an array type device.

Here, a single element (e.g., a single ultrasonic sensor) may refer to an ultrasonic sensor in which the number of all components including a piezoelectric material is one. In addition, an array-type element (e.g., an array ultrasonic sensor) may be an ultrasonic sensor in which the number of all components including a piezoelectric material is plural (n), and generally may be configured in a form mainly used in hospitals. In this case, the shape may be a linear shape, a convex shape, a 2D matrix, or the like.

In this example, similar to PMN-PT, it may be possible to manufacture both single or array ultrasonic sensors with small apertures.

The material characteristics of the piezoelectric layer 2151 may be summarized in Table 21 below.

**[Table 21]**

| | LNO | PMN-PT | PVDF & PVDF-TrFE |
|---|---|---|---|
| Size | Large | Small | Medium |
| Size of band width | Medium | Medium | broad |
| Available frequency range | 1∼400 MHz | 1∼100 MHz | 1kHz ∼100 MHz |
| Signal transmission performance | Good | Good | Bad |
| Signal reception performance | Good | Good | Good |
| Electric machine coupling coefficient | Medium | Good | Bad |

The first and second electrode layers 2153 and 2155 respectively located on the front and rear surfaces of the piezoelectric layer 2151 may receive a (+) drive signal and a (-) drive signal from a drive signal generator (not shown), respectively, and exhibit a converse piezoelectric effect on the piezoelectric layer 2151 so that an ultrasonic signal may be transmitted toward the sample A, and conversely, receive an electrical signal generated by the piezoelectric effect of the piezoelectric layer 2151 based on the ultrasonic signal received after being reflected by the sample A to be output externally.

The first and second electrode layers 2153 and 2155 may be formed of a transparent conductive material as described above, and may contain, for example, at least one of silver nanowire (AgNW), ITO, carbon nanotube, and graphene.

For easy coupling with the first housing 2171 and the second housing 2173, a size of the first electrode layer 2153 and a size of the second electrode layer 2155 may be different from each other.

Accordingly, in the first and second electrode layers 2153 and 2155 having a circular planar shape, a diameter of the second electrode layer 2155 is different from a diameter of the first electrode layer 2153, so that a portion (e.g., an edge portion) of the second electrode layer 2155 may be drawn out from the edge portion of the first electrode layer 2153.

When an electrical signal (e.g., a pulse signal) is applied to the piezoelectric material, the piezoelectric material (i.e., the piezoelectric layer 2151) vibrates back and forth to generate an ultrasonic signal, and the ultrasonic signal may also be generated even from a rear surface of the piezoelectric layer 2151 facing the sample A, as well as a front surface opposite to the rear surface.

At this time, since the ultrasonic signal generated from the rear surface is not transmitted toward the sample A, the ultrasonic signal generated from the rear surface acts as a noise signal. Also, a portion of the ultrasonic signal reflected by the sample A and returned may pass through the matching unit 215 and output toward the correction lens unit 219.

Therefore, the rear layer 216 is located on the rear surface of the piezoelectric unit 215 to damp the ultrasonic signal generated from the rear surface of the piezoelectric unit 215 and to damp the ultrasonic signal reflected by the sample A.

As such, since the rear layer 216 is located on the rear surface of the piezoelectric unit 215 (that is, the surface located opposite to the front surface of the piezoelectric unit 215 on which the reflected ultrasonic signal is incident), the incident ultrasonic signal does not pass through the rear surface of the piezoelectric unit 215.

Accordingly, unnecessary signal interference by the ultrasonic signal passing through the rear surface of the piezoelectric unit 215 may be prevented, and loss of the ultrasonic signal reflected by the piezoelectric unit 215 may be prevented, thereby reducing a ring down signal to reduce ring down phenomenon.

Ring down, as a phenomenon in which unnecessary signals are elongated in a time axis, is a factor adversely affecting image generation.

Accordingly, the rear layer 216 may be appropriately manufactured by adjusting at least one of acoustic impedance and thickness in order to reduce the ring-down phenomenon.

When the rear layer 216 is formed of a material having a high acoustic impedance, the ring-down phenomenon is reduced, and the reduction of the ring-down phenomenon on the time axis is similar to the meaning of widening of a bandwidth in a frequency domain. However, instead, a magnitude of the entire ultrasonic signal may also be damped by the rear layer 216 during transmission and reception of the ultrasonic signal.

Conversely, when the rear layer 216 is formed of a material having a relatively low acoustic impedance, the bandwidth may be reduced without significantly reducing the ring down phenomenon, but the amount of transmission and reception of ultrasonic signals may be increased.

The rear layer 216 may also be formed of a transparent non-conductive material, and may be formed of, for example, transparent epoxy (e.g., Epotek301) or transparent glass.

When the rear layer 216 is formed of Epotek301, if the acoustic impedance is as low as 3.1 Mrayl, low signal damping is achieved, so that the transparent ultrasonic sensor 20 may obtain a relatively high signal.

In addition, Epotek301 has a very high transparency, such as having a transparency of about 95% or more at a wavelength of 380 nm to 2000 nm and cured at room temperature, and thus, the rear layer 216 is easily manufactured.

When the rear layer 216 is formed of glass, transparency and flatness are high and a separate curing process is unnecessary.

When the glass has an acoustic impedance of about 213 Mrayl, a pulse length is reduced due to a high signal damping operation in the rear layer 216, which reduces the ring-down effect but exhibits the effect of increasing the bandwidth of the frequency of the transparent ultrasonic sensor 20.

The rear layer 216 may be omitted if necessary.

The first housing 2171 and the second housing 2173 are respectively connected to the first electrode layer 2153 and the second electrode layer 2155 as described above. Accordingly, the first housing 2171 and the second housing 2173 may be formed of a transparent conductive material containing a conductive material (e.g., copper) through which an electric signal is transmitted.

Accordingly, the first housing 2171 may receive a corresponding signal through a first signal line L1 (not shown) and transmit the received signal to the first electrode layer 2153, and conversely, may output a signal applied from the first electrode layer 2153 to the first signal line L1.

The second housing 2173 may also receive the corresponding signal through a second signal line L2 (not shown), which is a separate signal line from the first signal line L1, and transmits the received signal to the second electrode layer 2155, and conversely, may output from a signal applied from the second electrode layer 2155 to the second signal line L2.

In this example, the signal input to the first signal line L1 may be a pulse signal, and the signal flowing into the second signal line L2 may be a ground signal or a shield signal (-), so that the first housing 2171 may transfer the pulse signal to the first electrode layer 2153, and the second housing 2173 may transfer the ground signal to the second electrode layer 2155.

The first housing 2171 and the second housing 2173 may have a ring shape and may be located to be in contact with the edge portion of the corresponding electrode layers 2153 and 2155, respectively, in contact therewith, i.e., in contact with a circular side surface.

That is, the first electrode layer 2153 and the second electrode layer 2155 may be inserted and mounted into an empty space located inside the first housing 2171 and the second housing 2173.

Accordingly, as shown in FIG. 2, the first housing 2171 and the second housing 2173 may be located to surround an actual active region AR1 of the transparent ultrasonic sensor 20, thereby minimizing a reduction in the active region AR1 due to the first and second housings 2171 and 2173, substantially, the first housing 2171.

As described above, since the first housing 2171 and the second housing 2173 serve to transmit electrical signals to the corresponding electrode layers 2153 and 2155, the first housing 2171 and the second housing 2173 may contain a material having good conductivity.

Since the first housing 2171 is located at the edge portion of the first electrode layer 2151 located on the entire rear surface of the piezoelectric layer 2151 through which light is received, the first housing 2171 may have a width W11 as thin as possible and may have a thickness as thick as possible to minimize a signal loss rate due to wiring resistance or the like.

As shown in FIGS. 3 and 4, the second housing 2173 is coupled to the second electrode layer 2155 having a larger diameter than the first electrode layer 2153, and thus has a larger diameter than the first housing 2171.

In addition, since the second housing 2173 is located on an outer side than the first housing 2171 to serve to protect the transparent ultrasonic sensor 20, the second housing 2173 may have a width and thickness greater than the width and thickness of the first housing 2171.

Accordingly, the first electrode layer 2153 and the first housing 2171 may be located in the second housing 2173.

In addition, as already described, an outer surface of the second housing 2173 exposed to the outside is covered with the protective layer 211 to prevent a noise signal from being introduced into the transparent ultrasonic sensor 20 through the second housing 2173.

Since the second housing 2173 does not affect a light receiving area of the piezoelectric layer 2151, a size thereof may be increased as necessary.

In addition, a desired optical component may be coupled to the second housing 2173 by forming a screw thread or a connector on the second housing 2173. In this case, the second housing 2173 may function as a coupling portion for coupling with other components.

The insulating unit 218 may be located between the first housing 2171 and the second housing 2173 that transmits the corresponding electric signals to the corresponding electrode layers 2153 and 2155 and is located in contact with the corresponding housings 2171 and 2173 to insulate the first housing 2171 and the second housing 2173 to prevent an electric short or short and serve to fix the positions of the first housing 2171 and the second housing 2173.

The insulating unit 218 may be formed of a transparent insulating material, such as non-conductive epoxy. When the matching unit 213 uses a plano-concave acoustic lens as an example, a focus of light and an ultrasonic signal incident after being reflected from the sample A may be adjusted by the acoustic lens of the matching unit 213, but after passing through the matching unit 213, a light spreading phenomenon may occur.

Therefore, the correction lens unit 219, which has a plano-convex shape opposite to that of the acoustic lens used in the matching unit 213, is positioned in front of the back layer 217, compensates for this light refraction phenomenon and prevents light from spreading.

In this case, a curvature of the correction lens unit 219 may be selectively used according to a position at which light is finally positioned.

As such, the correction lens unit 219 affects only the focus of light regardless of the focus of the ultrasonic signal, but the acoustic lens of the matching unit 213 may affect both the focus of the ultrasonic signal and the focus of the light.

The correction lens unit 219 may be omitted if necessary, and a focal length of light may be adjusted by changing the correction lens unit 219.

In addition, the correction lens unit 219 may have a confocal function of simultaneously adjusting the focus of the reflected and received ultrasonic signal and the focus of the light. However, when the correction lens unit 219 has a confocal function, the correction lens unit 219 should be designed in consideration of the shape of light before passing through the transparent ultrasonic sensor 20.

In this example, the correction lens unit 219 includes a single lens, but is not limited thereto. In addition to a single lens, such as a plano-convex lens, the correction lens unit 219 may additionally include a lens for aberration correction to include a plurality of lenses.

When all the components located in the active region AR1 of the transparent ultrasonic sensor 20 having such a structure are formed of a transparent material through which light is transmitted, the transparent ultrasonic sensor 20 has the following characteristics:

First, since the optical impedance is matched, that is, matched by the operation of the matching unit 213, the reliability of a signal output from the transparent ultrasonic sensor 20 may be improved.

In addition, due to the use of the acoustic lens having a focus adjustment function used in the matching unit 213, the focus of light reflected by the sample A and the ultrasonic signal is adjusted so that light and the ultrasonic signal may be focused at an accurately desired position of the piezoelectric unit 215. Accordingly, the clarity of the ultrasound image obtained by the signal output from the transparent ultrasonic sensor 20 may be significantly improved, so that not only the presence of the corresponding sample A but also an accurate shape of the detected sample A may be recognized.

In addition, as already described, since the components (e.g., 211 to 216 and 219) constituting the transparent ultrasonic sensor 20 are all formed of transparent materials, such as transparent glass, transparent epoxy, and transparent silicone, light output from the light source 10 may directly pass through the transparent ultrasonic sensor 20 and be irradiated toward the corresponding sample A.

Accordingly, the arrangement of the optical system including the transparent ultrasonic sensor 20 is free and the utilization of the space in which the optical system is installed may be improved.

In addition, the correction lens unit 219 may be selectively used according to the user's needs, and the focal length of light may be adjusted by changing the correction lens unit 219.

In addition, when an optical lens having a plano-concave shape with a coating for 400 to 1000 nm is used as the acoustic lens according to the user's need, light may easily transmit at 400 to 1000 nm, so that the clarity of an ultrasound image may be improved. As a specific coating technique and thickness, a conventionally known coating technique may be widely applied within a range that meets the needs, and coating on the surface of the optical lens may be omitted.

When the optical lens having a plano-concave shape is used as the acoustic lens 213, the phenomenon of light spreading due to the acoustic lens may occur but may be supplemented by the correction lens unit 219 and a focus of light may be adjusted at a desired point. In this manner, the range of selection of the acoustic lens may be widened by the use of a compensating lens.

The shape of light is maintained by focusing adjustment by the acoustic lens 213 and the correction lens unit 219, and accordingly, a fine focus may be maintained, so that a high-resolution optical image (e.g., photoacoustic image or optical coherence tomography image) may be obtained.

In addition, the first and second signal lines L1 and L2 are connected to the first and second housings 2171 and 2173 constituting the housing of the transparent ultrasonic sensor 20, respectively, to apply an electrical signal to the first and second electrodes 2153 and 2155, the signal lines L1 and L2 may be easily connected.

Furthermore, by forming a screw thread 21731 or the like in the second housing 2173, which is an outer housing, connection or coupling with other optical elements may be facilitated. In this manner, since a required optical element is coupled to the second housing 2173 located at a portion completely unrelated to a path of light exit from the light source 10, light is normally incident on the piezoelectric unit 215 of the transparent ultrasonic sensor 20 without loss, and since light passes through the center of the transparent ultrasonic sensor 20 in a normal direction, light and an ultrasonic signal may be easily aligned.

Here, being perpendicular may mean that light travels in a direction perpendicular to an incident surface of the transparent ultrasonic sensor (e.g., the transparent ultrasonic sensor).

As such, when light is vertically incident on the ultrasonic sensor, the focal positions of the light and the ultrasonic signal may exactly match, and thus the clarity of an image obtained from the transparent ultrasonic sensor may be further improved.

As already described, a matching layer for minimizing ultrasonic energy loss in a medium due to a difference in acoustic impedance between the air and the medium may be present.

There may be more than one matching layer.

In a comparative example, the matching layer may be formed as follows.

When a medium of an ultrasonic signal is water or biological tissue (1.5 Mrayl), acoustic impedance matching is required for maximum transmission/reception efficiency of ultrasonic energy when a piezoelectric layer is LNO (34.5 Mrayl) or PMN-PT (37.1 Mrayl). In this case, more than one matching layer of material between 37.1 and 21.5 Mrayl may be required.

At this time, when a specific matching layer is generated using a KLM simulation tool (PiezoCAD, PZFLEX, etc.), a waveform of an ultrasonic signal transmitted from water or biological tissue should be checked through simulation to find a material of an appropriate matching layer, and since a thickness of the generated matching layer also significantly affects a waveform of the ultrasonic wave, the thickness of the matching layer needs to be adjusted to find an appropriate thickness. Theoretically, a thickness of a minimum loss of wave energy is a minimum loss at a desired thickness of V4 by a wave equation (c = λ*f, c: speed of sound about 21480 m/s, λ: wavelength, f: desired center frequency).

In a typical ultrasonic sensor, a first matching layer is often formed of a mixture (7.9 Mrayl) of silver powder and epoxy. At this time, it is possible to adjust acoustic impedance according to a mixing ratio of the silver powder and the epoxy, and, for example, silver powder: epoxy = 3:1.25.

Thereafter, a second matching layer may be generated by parylene (2.8 Mrayl) coating.

If the piezoelectric layer is PVDF or PVDF-TrFE (about 4 Mrayl), only parylene coating may be used to generate one matching layer. Here, the matching layer formed by parylene coating may serve not only as a matching layer but also as protection and insulation from the outside.

However, in the case of the transparent ultrasonic sensor 20 according to the present example, since the components (e.g., 211 to 216 and 219) located in the active region AR1 are transparent, the matching layer 213 may be formed using glass in the case of LNO or PMN-PT constituting the piezoelectric layer. At this time, it is slightly different depending on a raw material of glass (e.g. Borosilicate glass =13 Mrayl, crown glass=14.2 Mrayl, quartz=14.5 Mrayl, plate glass = 210.7 Mrayl, sodalime glass = 213 Mrayl), so desired glass may be appropriately selected and used.

Thereafter, a second matching layer (e.g., 2 to 6 Mrayl) may be generated using transparent epoxies or silicones (e.g., PDMS), and a third matching layer may be generated using parylene coating. In this case, generating of the second matching layer may be omitted and the second matching layer (e.g., 211) may be formed on the first matching layer (e.g., 213) directly using parylene coating. Also, in this case, a desired matching layer may be generated using a simulation waveform resulting from a KLM simulation.

In the transparent ultrasonic sensor 20 according to this example, as an example, an optical lens formed of borosilicate is used as the first matching layer, and a second matching layer is formed on the first matching layer through parylene coating, so that acoustic impedance matching and protection and signal isolation from the outside may be performed.

As already described, the optical lens may perform not only the function of acoustic impedance matching, but also focusing, that is, focusing the ultrasonic signal generated from the piezoelectric layer.

Since the transparent ultrasonic sensor 20 is mainly used for image acquisition, focusing of an ultrasonic signal is a factor which significantly affects high resolution and high sensitivity.

### [Detailed Description of Main Elements]

1: transparent ultrasonic sensor-based ultrasonic optical composite imaging system
10: light source
12: light source selecting unit
20: TUT
30, 32, 34: optical system
40, 42: mechanical scanner
50, 52: optical scanner
211: protective layer
213: matching unit
215: piezoelectric unit
2151: piezoelectric layer
2153: first electrode layer
2155: second electrode layer
216: rear layer
2171: first housing
2173: second housing
218: insulating unit
219: correction lens
A: sample

## Claims

1. A photoacoustic detector system combined with transparent ultrasonic sensors, comprising:
a light source from which an optical oscillation device generates light appropriate for generating a photoacoustic phenomenon;
a light transmission system which transmits the light generated from the light source to a probe side;
a probe which accommodates an optical system and the transparent ultrasonic sensor therein;
the optical system which adjusts the size, focus, path, etc., of the light generated from the light source;
the transparent ultrasonic sensor which detects an ultrasonic wave generated in an observation area in response to light irradiated to the observation area according to a photoacoustic effect, and is made of a light-transmitting material to transmit the light generated from the light source;
a data collection device which collects photoacoustic data including the ultrasonic wave detected by the transparent ultrasonic sensor; and
a data detection device which analyzes the photoacoustic data and determines whether a specific material is detected in the observation area.

2. The photoacoustic detector system combined with transparent ultrasonic sensors of claim 1, wherein the light source is at least one light oscillation device selected from the group at least including n optically mediated resonator laser (OPO laser), a liquid die laser, an LED, an LD, a solid die laser, and an alexandrite laser.

3. The photoacoustic detector system combined with transparent ultrasonic sensors of claim 1, wherein the light transmission system includes at least one of an optical fiber and an articulated arm.

4. The photoacoustic detector system combined with transparent ultrasonic sensors of claim 1, wherein the optical system at least includes a beam expander, a mirror, and a lens.

5. The photoacoustic detector system combined with transparent ultrasonic sensors of claim 1, wherein the transparent ultrasonic sensor is any one of a focus type and a non-focus type.

6. The photoacoustic detector system combined with transparent ultrasonic sensors of claim 1, wherein the transparent ultrasonic sensor is a single transparent ultrasonic sensor element.

7. The photoacoustic detector system combined with transparent ultrasonic sensors of claim 1, wherein the transparent ultrasonic sensor is constituted by an array of a plurality of transparent ultrasonic sensor elements.

8. The photoacoustic detector system combined with transparent ultrasonic sensors of claim 1, wherein a path axis of the light and a path axis of the ultrasonic wave are formed parallel to each other.

9. The photoacoustic detector system combined with transparent ultrasonic sensors of claim 1, further comprising:
a wavelength conversion system which changes the wavelength of the light.

10. The photoacoustic detector system combined with transparent ultrasonic sensors of claim 1, further comprising:
a medium capable of transmitting the ultrasonic wave between the transparent ultrasonic sensor and the observation area.

11. The photoacoustic detector system combined with transparent ultrasonic sensors of claim 1, wherein the data collection device receives the optoacoustic data by being wired or wirelessly connected to the transparent ultrasonic sensor.
